# EUROPEAN PATENT SPECIFICATION

(11) **EP 4 273 247 B1**
(45) Date of publication and mention of the grant of the patent: **23.09.2026**
(21) Application number: 21913020.0
(22) Date of filing: 14.07.2021
(51) Int. Cl.: C12N 15/51, C12N 5/10

(54) **NUCLEIC ACID, VECTOR AND HOST CELL FOR PREPARING HBV CCCDNA**
NUKLEINSÄURE, VEKTOR UND WIRTSZELLE ZUR HERSTELLUNG VON HBV CCCDNA
ACIDE NUCLÉIQUE, VECTEUR ET CELLULE HÔTE POUR LA PRÉPARATION D'ADNCCC DE VHB

(30) Priority: 30.12.2020 CN 202011615935
(43) Date of publication of application: 08.11.2023
(73) Proprietor: Guangzhou Eighth People's Hospital Guangzhou Medical University, Guangzhou, Guangdong 510443 (CN)
(72) Inventor: LI, Feng, Guangzhou, Guangdong 510443 (CN); FENG, Chengqian, Guangzhou, Guangdong 510443 (CN)
(74) Representative: J A Kemp LLP
(86) International application number: PCT/CN2021/106139
(87) International publication number: WO 2022/142267

(56) References cited:
- CN-A- 107 208 102
- CN-A- 109 897 825
- CN-A- 110 592 026
- US-A1- 2020 377 892
- ZHIHUA QI ET AL: "Recombinant Covalently Closed Circular Hepatitis B Virus DNA Induces Prolonged Viral Persistence in Immunocompetent Mice", JOURNAL OF VIROLOGY, vol. 88, no. 14, 15 July 2014 (2014-07-15), US, pages 8045 - 8056, XP055268374, ISSN: 0022-538X, DOI: 10.1128/JVI.01024-14
- LI FENG ET AL: "Minicircle HBV cccDNA with a Gaussia luciferase reporter for investigating HBV cccDNA biology and developing cccDNA-targeting drugs", vol. 6, no. 1, 7 November 2016 (2016-11-07), US, XP093262609, ISSN: 2045-2322, Retrieved from the Internet <URL:https://www.nature.com/articles/srep36483> DOI: 10.1038/srep36483
- YAN ZHIPENG ET AL: "HBVcircle: A novel tool to investigate hepatitis B virus covalently closed circular DNA", JOURNAL OF HEPATOLOGY, ELSEVIER, AMSTERDAM, NL, vol. 66, no. 6, 14 February 2017 (2017-02-14), pages 1149 - 1157, XP085023478, ISSN: 0168-8278, DOI: 10.1016/J.JHEP.2017.02.004
- BI YANWEI, ET AL.: "Recent Advances in HBV Research Models", CARCINOGENESIS, TERATOGENESIS & MUTAGENESIS, vol. 31, no. 2, 28 March 2019 (2019-03-28), pages 166 - 170, XP055948584, ISSN: 1004-616X, DOI: 10.3969/j.issn.1004-616x.2019.02.014
- ZHU YUANFEI, ET AL.: "Preparing Recombinant cccDNA of Hepatitis B Virus in Vitro Using Minicircle DNA Vector Technology", JOURNAL OF MICROBES AND INFECTIONS, vol. 12, no. 4, 25 August 2017 (2017-08-25), pages 229 - 234, XP055948581, ISSN: 1673-6184
- GUO XIAOYAN, CHEN PING, HOU XIAOHU, XU WENJUAN, WANG DAN, WANG TIAN-YAN, ZHANG LIPING, ZHENG GANG, GAO ZHI-LIANG, HE CHENG-YI, ZHO: "The recombined cccDNA produced using minicircle technology mimicked HBV genome in structure and function closely", SCIENTIFIC REPORTS, vol. 6, no. 1, 1 May 2016 (2016-05-01), XP055948579, DOI: 10.1038/srep25552

## Description

### TECHNICAL FIELD

The present disclosure relates to the technical field of genetic engineering, in particular to a nucleic acid, a vector, and a host cell for preparing HBV cccDNA.

### BACKGROUND

Chronic hepatitis B is a disease caused by infection with hepatitis B virus (HBV). HBV is a DNA virus belonging to the family Hepadnaviridae.The virus DNA enters the host cell nucleus during the replication of hepatitis B virus. Under the action of DNA polymerase, the gaps of both chains are filled to form a supercoiled covalently closed circular DNA molecule (cccDNA). cccDNA, which is the original template for the replication of hepatitis B virus pregenomic RNA, is no more than 50 copies in each liver cell in the patient's liver tissue even at the most active stage of virus replication. In spite of a small amount, HBV cccDNA as a template for producing new viruses is of great significance for the replication of hepatitis B virus, the establishment of infection states, and the reactivation of viruses in the course of clinical treatment. Only by completely eliminating the HBV cccDNA in the cell nucleus can the HBV infection and carrier state be completely cured, resulting in a complete cure for hepatitis B patients, which is the ultimate goal of antiviral therapy.

Currently, two regimens, i.e., nucleoside analogs (NAs) and interferon (IFN), are generally used in the clinical treatment. Nucleoside analogs can effectively inhibit the replication and reverse transcription process of viruses and prevent the generation of new viruses. However, nucleoside analogs cannot directly reduce HBV cccDNA because they are not designed to directly target cccDNA. Even after years of antiviral treatment, the serological conversion rate of HBV antigens would not exceed 20%. The challenge of occurrence and spreading of drug-resistant strains are also clinically encountered. This is mainly due to the non-strict pairing of RNA and DNA in reverse transcription during the generation of new HBV viruses, which led to a high mutation rate, and eventually, the prevalence of drug-resistant strains that develops resistance to nucleoside analog drugs. Interferon, which is a major class of immunomodulatory drugs, is applied in the field of HBV treatment based on the fact that interferon has extensive antiviral activity. However, the mechanism of interferon action is not clear, resulting in a relatively limited clinical application. Interferon is not suitable for all HBV-infected patients, while it has a good cure rate in special populations. The detailed action mechanism of interferon is still under study, and it is also a hot spot in the field of HBV treatment.

At present, most knowledge about cccDNA is based on the duck model of DHBV infection. However, as the human cccDNA greatly differs from duck cccDNA in biological characteristics, the knowledge obtained by using DHBV as a model has very limited practical guiding significance for researching human HBV-cccDNA. In recent years, the cell line models for cccDNA research mainly comprise two categories, one of which is a cell line that is infected with pseudovirus carrying HBV gene and forms cccDNA through transcription and expression; the other is a cell line in which cccDNA is intracellularly formed by a series of molecular biological means. However, both cell lines are low-efficient in term of the formation of cccDNA, and cannot well simulate the true state of the cccDNA production process in vivo.

Zhihua Qi et al., J. Virol. (2014) 88(14):8045-8056, Li Feng et al., Scientific Reports (2016 6:36483, and Yan Zhipeng et al., J. Hepatol. (2017) 66(6):1149-1157 teach different methods of producing HBV recombinant cccDNA.

### SUMMARY

Accordingly, the present disclosure provides a nucleic acid for preparing HBV cccDNA, which can improve the efficiency of HBV cccDNA formation in cells.

In addition, it is necessary to provide a vector and a host cell capable of improving the efficiency of HBV cccDNA formation.

A nucleic acid for preparing HBV cccDNA includes a first segment and a second segment linked to the first segment. The first segment and the second segment are each transcribed independently. The first segment comprises a Core gene encoding a core protein, a Pol gene encoding an HBV polymerase and an X gene encoding an HBx. The second segment comprises a nucleic acid fragment corresponding to a pregenomic RNA and a nucleic acid fragment corresponding to a start codon; and the nucleic acid fragment corresponding to the start codon is positioned at the 3' end of the transcription direction of the nucleic acid fragment corresponding to the pregenomic RNA. Only one nucleic acid fragment corresponding to the start codon is present in the second segment; and the nucleic acid contains no expression frame of S gene encoding an HBs protein.

In the nucleic acid for preparing HBV cccDNA as described above, the first segment is configured to express the HBV core protein, the HBV polymerase and the HBx. The HBx expressed in the first segment can improve the transcription activity of the HBV cccDNA and greatly improve the expression level of genes carried by the HBV cccDNA. The HBV core protein and the HBV polymerase expressed in the first segment can guarantee the HBV pregenomic RNA (pre-RNA (pgRNA) produced in the second segment being reverse transcribed into HBV cccDNA. Meanwhile, since the nucleic acid fragment corresponding to the start codon at the 3' end of the second segment is located at the 5' end of the nucleic acid fragment corresponding to the pregenomic RNA in the formed circular HBV cccDNA upon the formation of the circular HBV cccDNA, the formed HBV cccDNA can be transcribed into a transcript with AUG at the 5' end, and in turn, the viral RNA sequence can be translated into a complete protein.

In addition, in the HBV viral replication cycle, HBc first forms an empty capsid, in which HBVpgRNA is reverse transcribed via HBVPol to produce rcDNA, forming mature nucleocapsids. The mature nucleocapsid thereafter have two destinations. Destination 1 is the extracellular secretory pathway which is the main destination of the mature nucleocapsids. The mature nucleocapsids and the HBsAg on the endoplasmic reticulum form complete virus particles with envelopes, which are released in a budding manner. Destination 2 is the internal circulation pathway, which is a secondary destination accounting for a relatively small proportion. The mature nucleocapsids form cccDNA in the nucleus through a pathway that has not yet been fully understood. In the absence of HBsAg, the extracellular secretory pathway is blocked, and all mature nucleocapsids are changed to the internal circulation pathway, which can greatly increase the level of HBV cccDNA in the nucleus. Therefore, by designing the nucleic acid for preparing the HBV cccDNA to contain no expression frame of S gene encoding an HBs protein, the coding frames of the surface protein (HBs) of the HBV in both the first segment and the second segment are inactivated, thereby avoiding the production of the HBsAg during the production of the cccDNA, and improving the production level of the cccDNA.

In addition, when the above nucleic acid for preparing the HBV cccDNAis used to prepare the HBV cccDNA, the generation of the cccDNA does not depend on infection with the HBV virus, but avoids the generation of the infectious virus, allowing the research work to be carried out in conventional P2 laboratories and extending its application scenarios.

In an embodiment, the second segment further comprises a reporter gene. The reporter gene, the nucleic acid fragment corresponding to the pregenomic RNA and the nucleic acid fragment corresponding to the start codon are sequentially linked in the transcription direction of the second segment.

In an embodiment, the reporter gene is at least one selected from the group consisting of a luciferase gene, a chloramphenicol acetyltransferase gene, a beta-galactosidase gene, a secretory human placental alkaline phosphatase gene, and a green fluorescent protein gene.

In an embodiment, the Core gene has a nucleotide sequence as set forth in SEQ ID NO: 1; and/or
the Pol gene has a nucleotide sequence as set forth in SEQ ID NO: 2; and/or
the X gene has a nucleotide sequence as set forth in SEQ ID NO: 3.

In an embodiment, the first segment further comprises an antibiotic resistance gene. The antibiotic resistance gene is linked to the Core gene via a nucleic acid fragment encoding a 2A peptide; optionally, the antibiotic resistance gene has a nucleotide sequence as set forth in SEQ ID NO: 5.

In an embodiment, the first segment has a nucleotide sequence as set forth in SEQ ID NO: 6; and/or
the second segment has a nucleotide sequence as set forth in SEQ ID NO: 7.

A vector for preparing HBV cccDNA includes the nucleic acid for preparing HBV cccDNA as described above.

A host cell includes the vector for preparing HBV cccDNA as described above.

A method for preparing HBV cccDNA includes the following steps:
transfecting a host cell with a vector for preparing the HBV cccDNA as described above and then culturing the host cell to construct a stable cell line for preparing HBV cccDNA.

In an embodiment, the host cell is HepG2, Huh7, a cell line derived from HepG2 or a cell line derived from Huh7.

### BRIEF DESCRIPTION OF ACCOMPANYING DRAWINGS

FIG. 1 is a schematic diagram of the principle of preparing cccDNA by the nucleic acid for preparing HBV cccDNA in an embodiment.
FIG. 2 is a map of a plasmid for preparing HBV cccDNA in Example 1.
FIG. 3 shows the results of screening and identification of HepG2 stable cell lines in Example 1.
FIG. 4 shows the identification results of the cccDNA prepared in Example 1 by PCR.
FIG. 5 shows the results of the drug evaluation in Example 1.

### DETAILED DESCRIPTION

To make the present disclosure easy to understand, a more comprehensive description of the present disclosure will be given below. The various embodiments of the disclosure may, however, be embodied in many different forms and should not be construed as limited to the embodiments set forth herein. Rather, these embodiments are provided so that this disclosure will be thorough and complete.

Unless otherwise defined, all technical and scientific terms used herein have the same meanings as those generally understood by those skilled in the art of the present disclosure. The terms used in the specification of the present disclosure are only intended to describe specific embodiments, rather than limiting the present disclosure. Additionally, it should be noted that "HBc" or "HBcAg" herein refers to the core antigen of HBV; As used herein, "HBs" or "HBsAg" refers to the surface antigen of HBV.

The genome of HBV is a partially double-stranded DNA molecule with a length of about 3200 bp. The two linear DNA strands form a circular structure through a 226-bp sticky end between the 5' ends. There are direct repeat sequences consisting of 11 bp at both sides of the sticky end, called DR1 and DR2. The HBV genes can be guided by DR1 and integrated into the genome of a host, so that the HBV genes can be continuously formed along with the proliferation of the host.

The negative strand of HBV contains 4 open reading frames (ORFs), namely Pre-S/S ORF (referred to as "S region"), Pre-C/C ORF (referred to as "C region"), Pol ORF (referred to as "P region") and X ORF (referred to as "X region"). The S region, which is mainly responsible for encoding an outer capsid (equivalent to an envelope of a common virus), includes an S gene, a pre-S1 gene and a pre-S2 gene. The S gene encodes the outer capsid protein HBs of HBV, the pre-S1 gene encodes a Pre S1 antigen, and the pre-S2 gene encodes a PreS2 antigen. The C region, which is mainly responsible for encoding an inner capsid (equivalent to a nucleocapsid of a common virus), includes a C gene and a pre-C gene. The C gene encodes the core protein HBc, and the pre-C gene encodes HBe. The P region is mainly responsible for encoding the Pol gene, which encodes HBV polymerase (pol). The P region is the longest ORF, spanning 80% of the entire genome and overlapping with the other three ORFs. The X region has an X gene, which encodes HBx.

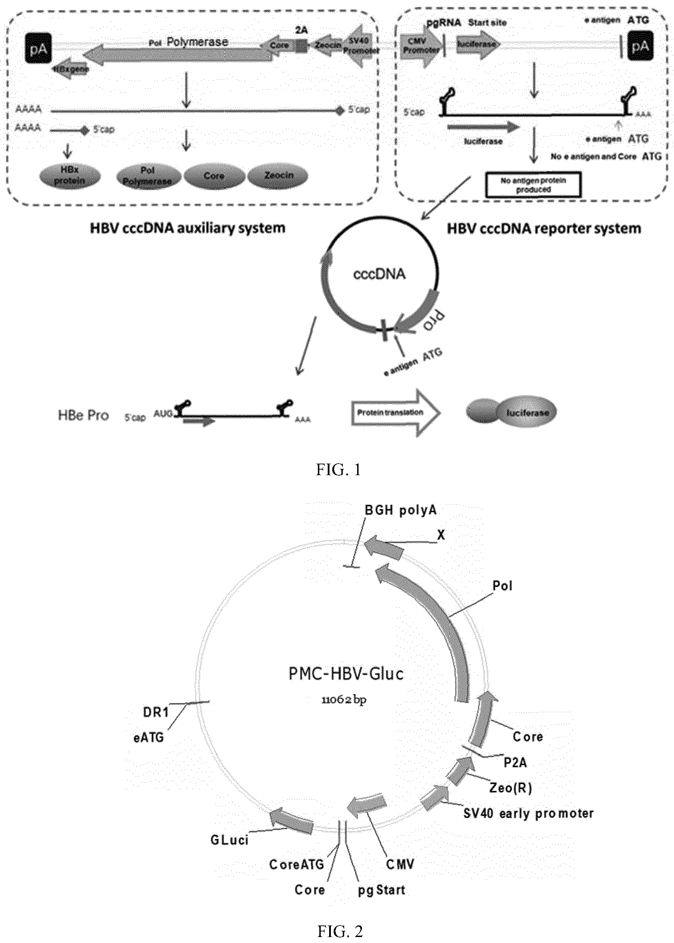

Referring to FIG. 1, an embodiment of the present disclosure provides a nucleic acid for preparing HBV cccDNA including a first segment and a second segment linked to the first segment. The first segment and the second segment are each transcribed independently and in opposite directions. The first segment comprises a Core gene encoding a core protein, a Pol gene encoding an HBV polymerase and an X gene encoding an HBx. The second segment comprises a nucleic acid fragment corresponding to a pregenomic RNA and a nucleic acid fragment corresponding to a start codon. The nucleic acid fragment corresponding to the start codon is positioned at the 3' end of the transcription direction of the nucleic acid fragment corresponding to the pregenomic RNA. Only one nucleic acid fragment corresponding to the start codon is present in the second segment; and the nucleic acid for preparing the HBV cccDNA contains no expression frame of S gene encoding an HBs protein.

The first segment is mainly configured to produce cofactors such as core proteins, HBV polymerase and HBx that assist cccDNA formation. In particular, the first segment includes a Core gene encoding a core protein, a Pol gene encoding an HBV polymerase and an X gene encoding HBx.

The HBV core protein (HBc) encoded by the Core gene is the main protein of the HBV nucleocapsid. Multiple HBcs form a closed HBV nucleocapsid which encloses HBV pgRNA, HBVPol and a part of cell components, providing a place and raw materials for the reverse transcription of HBV pgRNA into HBV virus genome (i.e., replicative circular DNA, rcDNA). Depending on the HBV genotype, HBc consists of 183,185 or 195 amino acids. HBc contains two completely different functional regions, including an assembling region of the 32-nm inner capsid consisted of amino acids 1-144, and an arginine-rich region from the carboxyl end of HBc to amino acid 150, which can bind to nucleic acid and is related to the packaging and replication of HBV. The arginine-rich region at the carboxy end of HBc can be further divided into four arginine cluster regions and further contains a nuclear localization sequence.

In this embodiment, the Core gene has a nucleotide sequence as set forth in SEQ ID NO: 1.

The translation of the Pol gene starts from HBV pregenomic RNA, yielding a product that is a multi-functional protein (HBV-polymerase) with 844 amino acids including four functional regions: a terminal protein (TP) at the amino end, which can covalently bind to the 5' end of the HBV negative chain to guide the synthesis of the HBV negative chain; a spacer region next to the TP, which has not been found to have special functions; an RNase H region located at the carboxyl end of the HBV polymer, which can cleave RNA in an RNA-DNA hybrid formed during reverse transcription; and a reverse transcriptase region (RT) between the spacer region and RNase H region, which is RNA and DNA-dependent DNA polymerase that catalyzes reverse transcription of HBV and synthesis of DNA strands. HBV polymerase is also an important structural protein in addition to its role as a functional protein in HBV replication. A complete HBV polymerase is necessary for the packaging of HBV pregenomic RNA.

In this embodiment, the Pol gene has a nucleotide sequence as set forth in SEQ ID NO: 2.

HBx encoded by the X gene is another accessory protein of HBV. The expression of full-length HBx is not a necessary component in the process of HBV proliferation in vitro, but it is a key factor in the process of in vivo infection. HBx protein transactivates promoters of HBV and cellular genes by directly interacting with transcription factors, such as RPB5 subunit of RNA polymerase II, TATA binding protein, ATB, etc. In this embodiment, the first segment retains the ability of HBx to express the HBx protein and may increase the transcriptional activity of the HBV cccDNA, thereby greatly increasing the expression level of genes carried by the HBV cccDNA.

In this embodiment, the X gene has a nucleotide sequence as set forth in SEQ ID NO: 3.

Optionally, in the first segment, the Core gene encoding the core protein, the Pol gene encoding the HBV polymerase and the X gene encoding HBx are sequentially linked in the transcription direction of the first segment. Of course, in other embodiments, the order for linking the Core gene, the Pol gene, and the X gene is not limited, as long as the corresponding protein can be produced.

In some embodiments, the first segment also includes an antibiotic resistance gene. The antibiotic resistance gene facilitates the screening for the segments that are correctly linked. In particular, the antibiotic resistance gene is located at the beginning of transcription of the first segment and is closer to the promoter than the other genes. Optionally, the antibiotic resistance gene, the Core gene, the Pol gene, and the X gene are sequentially linked in the transcription direction of the first segment. Further, the antibiotic resistance gene is linked to one of the other genes in the first segment via a nucleic acid fragment encoding a 2A peptide (self-cleaving peptide). The antibiotic resistance gene that is fused with other genes in the first segment is cleaved by the 2A peptide upon expression into an independent protein. In an optional specific example, the antibiotic resistance gene is linked to the Core gene via a nucleic acid fragment encoding the 2A peptide. The antibiotic resistance gene has a nucleotide sequence as set forth in SEQ ID NO: 5.

In this embodiment, the first segment has a nucleotide sequence as set forth in SEQ ID NO: 6.

The second segment comprises a nucleic acid fragment corresponding to a pregenomic RNA and a nucleic acid fragment corresponding to a start codon. The nucleic acid fragment corresponding to the start codon is positioned at the 3' end of the transcription direction of the nucleic acid fragment corresponding to the pregenomic RNA. The second segment is configured to be transcribed into the pregenomic RNA (pgRNA) to form HBV. The pgRNA, which contains all the genetic information of HBV, is a template for reverse transcription. Under the action of HBV polymerase, the pgRNA is reverse transcribed into the negative strand of DNA of HBV. Then, the positive strand is synthesized by using the negative strand as the template to form an rcDNA. The double strands are circularized to form the cccDNA.

HBe is a secreted form of HBc protein that is soluble and is considered an accessory protein of HBV. The pre-C gene is located upstream of the gene sequence of the core protein. A 212-amino acid pre-C/C protein (P25), which is the precursor protein of HBe and consisting of a core protein and 29 amino acids at the amino end, is synthesized by starting from the AUG of the pre-C gene. The 19 amino acids at the beginning of P25 act as a signal peptide, providing signals for the transport of P25 into the endoplasmic reticulum. The signal peptide is then cleaved by a signal peptidase of the host to form an intermediate P22. The 34 amino acids at the carboxyl end of P22 are cleaved by a cellular protease to form a secretable protein of 15 kDa to 18 kDa, namely HBe.

In the second segment, only one nucleic acid fragment corresponding to the start codon is present, and this nucleic acid fragment corresponding to the start codon is positioned at the 3' end of the transcription direction of the nucleic acid fragment corresponding to the pregenomic RNA. By providing only one nucleic acid fragment corresponding to the start codon in the second segment, which is positioned at the 3' end of the transcription direction of the nucleic acid fragment corresponding to the pregenomic RNA, the mRNA formed by the transcription of the second segment has only one translation start site which is positioned at the 3' end of the mRNA, thus the protein at the 5' end cannot be translated. At this time, no protein is produced, and resources in the cell are focused on supporting pgRNA to be reverse transcribed into the negative chain of DNA of HBV, thereby promoting the formation of cccDNA.

Further, the second segment further comprises a reporter gene. The reporter gene, the nucleic acid fragment corresponding to the HBV pregenomic RNA and the nucleic acid fragment corresponding to the start codon are sequentially linked in the transcription direction of the second segment. Since the second segment contains only one nucleic acid fragment corresponding to the start codon and positioned at the 3' end of the transcription direction of the second segment, only after the cccDNA is synthesized, the mRNA that is transcribed using the negative strand of the cccDNA as a template can allow the expression of HBV genome proteins and related reporter proteins. Therefore, the reporter gene is configured to report the synthesis of cccDNA, and the state of the cccDNA in the cell can be simply and intuitively determined by the reporter gene.

In particular, the reporter gene is at least one selected from the group consisting of luciferase gene, chloramphenicol acetyltransferase gene, beta-galactosidase gene, secretory human placental alkaline phosphatase gene, and green fluorescent protein gene. Definitely, the reporter gene is not limited to the above, but may also be other reporter genes.

In this embodiment, the reporter gene is luciferase reporter gene (Gaussia Luciferase), and the reporter gene has a nucleotide sequence as set forth in SEQ ID NO: 4.

Optionally, the genotype of HBV is a strain prevalent in China, genotype C.

In this embodiment, the second segment has a nucleotide sequence as set forth in SEQ ID NO: 7.

In some embodiments, at least one of the first segment or the second segment further comprises an enhancer.

The nucleic acid for preparing the HBV cccDNA is designed to contain no expression frame of S gene encoding the HBs protein, so that no HBs would be generated, preventing the nucleocapsid containing the rcDNA from forming mature viruses and being secreted outside the cells, thereby increasing the level of endonuclear cccDNA formed from rcDNA and increasing the efficiency of cccDNA formation. Further, the nucleic acid fragment corresponding to the pregenomic RNA in the second segment does not contain an expression frame of S gene encoding the HBs protein.

From the nucleic acid for preparing the HBV cccDNA, the HBV polymerase (Pol), the HBe protein and the HBx protein are generated by the transcription and translation of the first segment, activates and assists the transcription of the second segment to form the HBV pregenomic RNA. Under the action of HBV polymerase, the HBV pregenomic RNA is reverse transcribed into HBV DNA negative strand, which further forms cccDNA. By deleting the expression frame of the S gene in the HBV genome in the second segment, the cccDNA produced would not only allow no mature viruses to be formed and secreted outside the cells but also continue to enter the nucleus for transcription. Thus, the intracellular cccDNA increases exponentially, resulting in high efficiency in preparing cccDNA. Further, the DR1 contained in the second segment allows the genomic DNA of HBV to be integrated into the genome of the host, and thereby to replicate along with the replication of the host cell.

Further, an embodiment of the present disclosure further provides a vector for preparing HBV cccDNA including the nucleic acid for preparing HBV cccDNA as described above.

Specifically, a vector for preparing HBV cccDNA includes an empty vector and a nucleic acid for preparing HBV cccDNA inserted into the empty vector. Optionally, the empty vector includes elements for transcription of the nucleic acid as described above, such as promoters, terminators and the like.

The above-mentioned vector for preparing HBV cccDNA has high efficiency when used for preparing HBV cccDNA due to the inclusion of the above-mentioned nucleic acid for preparing HBV cccDNA.

An embodiment of the present disclosure further provides a host cell includes the vector for preparing HBV cccDNA as described above.

Optionally, the host cell is a HepG2 cell containing the vector for preparing HBV cccDNA as described above. HepG2 cells have a simple background and are a good carrier for studying hepatitis B virus and cccDNA.

The above-mentioned host cell has high efficiency in producing HBV cccDNA and is a non-infected, quantifiable cell line that allows the direct formation of cccDNA in cells.

In addition, an embodiment of the present disclosure provides a method for preparing HBV cccDNA including the following steps:

transfecting a host cell with a vector for preparing the HBV cccDNA as described above and then culturing the host cell for preparing the HBV cccDNA.

Optionally, the host cell is a HepG2 cell. Definitely, in other embodiments, the host cell can be other liver-derived cell lines, such as Huh7, a cell line derived from hepg2, a cell line derived from Huh7, or the like.

The method for preparing HBV cccDNA as described above is simple and convenient with high efficiency in producing HBV cccDNA. The preparation method does not involve the generation of the virus, thus is safe and easy to operate, and can be used as a good platform for researching the formation mechanism of cccDNA and drug targets.

### Examples

The following will be described in detail in conjunction with specific examples. The following examples do not include other components except unavoidable impurities unless otherwise specified. The reagents and instruments used in the examples are all conventional choices in the art unless otherwise specified. The experimental methods for which specific conditions are not indicated in the examples are implemented according to conventional conditions, such as the conditions described in literature, books, or the method recommended by the manufacturer.

### Example 1

The vector for preparing HBV cccDNA in Example 1 has a structure as shown in FIG. 2, and was prepared as follows:

### 1. Obtaining First and Second Segments

All DNA fragments of the first and second segments required for the construction of the vector were respectively synthesized using the gene synthesis method (Nanjing GenScript). The information of the first and second segments is shown in FIG. 1. The first segment had a nucleotide sequence as set forth in SEQ ID NO: 6; and the second segment had a nucleotide sequence as set forth in SEQ ID NO: 7.

The first segment included four main gene fragments, Zeocin, Core, Pol, and X, and two restriction sites, NheI and HindIII, which were introduced at the 5' end. The second segment mainly included a nucleic acid fragment corresponding to the pregenomic RNA and a fluorescence reporter gene for Gaussia luciferase. Similarly, two cleavage sites, HindIII and NheI, were introduced at both ends.

The synthesized DNA fragments of the first segment and the second segment were ligated to a T cloning vector (Takata, Code No: 6011), indicated as PMC-Zeo-Core-Pol-HBx, and PMC-Gaussia luciferase.

### 2. Obtaining Complete Plasmid PMC-HBV-Gluc

(1) PMC-Zeo-Core-Pol-HBx as the backbone vector and PMC-GaussiaLuciferase as the target fragment were each digested with restriction endonuclease from TaKaRa Company in the reaction system as shown in Table 1.

**Table 1**

| Reagents | Volume |
|---|---|
| Plasmid | 10 µg |
| 10 x M Buffer | 2 µL |
| HindIII | 1 µL |
| NheI | 1 µL |
| RNase-Free ddH₂O | to 20 µL |

The target fragments were recovered from the digested products using Agarose Gel Recovery kit (Tiangen Biochemical Technology). In particular, the PCR products were separated in the agarose gel by molecular weight. A single target DNA band was cut out from the agarose gel (cut off the excess part as much as possible), put into a clean centrifuge tube, and weighed. To the gel block, an equal volume of solution PN was added. For a gel block with a weight of 0.1 g, its volume can be regarded as 100 µL, thus 100 µL PN solution would be added. After adding the solution PN, the centrifuge tube was placed in a water bath at 60 °C and gently inverted repeatedly to ensure that the gel block was sufficiently dissolved. The solution obtained in the previous step was added into an adsorption column CA2 (column equilibration was carried out by adding 500 µL of equilibrium solution BL to the adsorption column CA2, centrifuging at 12,000 rpm for 1 min, discarding the waste liquid in the collection tube, and placing the adsorption column into the collection tube). The adsorption column CA2 was left at room temperature for 2 min, and centrifuged at 12,000 rpm (~13,400 x g) for 30 s. The waste liquid in the collection tube was discarded, and the adsorption column CA2 was placed into the collection tube. 600 µL of rinsing solution PW (whether absolute ethanol has been added would be checked before use) was added to the adsorption column CA2, which was then centrifuged at 12,000 rpm (~13,400 x g) for 30 s. The waste liquid in the collection tube was discarded, and the adsorption column CA2 was placed into the collection tube. The collection tube was centrifuged at 12,000 rpm (~13,400 x g) for 2 min to remove the rinsing solution as much as possible. The adsorption column CA2 was left at room temperature for several minutes for drying thoroughly in order to prevent the remaining rinsing solution from affecting the following experiment. The adsorption column CA2 was placed in a clean centrifugal tube. A proper amount of elution buffer EB was untouchably dropwise added to the center of the adsorption membrane. The centrifugal tube was left at room temperature for 2 min. The centrifugal tube was centrifuged at 12,000 rpm (~13,400 x g) for 2 min to collect DNA solution.

(2) The PMC-Zeo-Core-Pol-HBx and Gaussia Luciferase were ligated for 8 hours using the SolutionI ligation kit from TaKaRa Company in a system as shown in Table 2 below.

**Table 2**

| Reagents | Volume |
|---|---|
| Solution I | 5 µL |
| PMC-Zeo-Core-Pol-HBx | 0.5 µL |
| Gaussia Luciferase | 4.5 µL |

(3) After completion of the reaction, the ligation product was transformed into TOP10 competent cells (Tiangen Biochemical Company), and the correct clones were identified by colony PCR and enzyme digestion to obtain the complete plasmid PMC-HBV-Gluc.

### 3. Transfecting HepG2 cell line with PMC-HBV-Gluc

HepG2 cells were seeded in a 10-cm culture dish, and transfected when the cell density reached 30%. The culture medium was replaced with fresh medium (Gibco DMEM+10%FBS) before transfection. Lipo 3000 was used as transfection reagent. PMC-HBV-Gluc and Lipo 3000 were diluted with Opti-MEM, thoroughly mixed and left standing at room temperature for 15 minutes, and then gently added to the culture medium in the cell dish without changing the culture medium. After 48 hours, the cell status was observed and positive cell was screened by adding the antibiotic Zeocin (sigma, 400 µg/mL).

### 4. Obtaining Monoclonal Cell Strain HepG2-HBV-Gluc

After 4 weeks of Zeocin screening, the cells that did not develop resistance would gradually be apoptotic, while the remaining positive cells would slowly divide and aggregate to form cell clones of different sizes. Once the cell clone grew to a certain size, a clone can be picked. The original medium in the 10 cm culture dish was replaced with a serum-free medium. The cell clone was cut off along its edge using a glass needle under the microscope, and then pipetted into an EP tube with 0.25% trypsin, digested for 2 minutes, and finally transferred to a 96-well culture plate for extended culture.

### 5. Identification of Monoclonal Cell Strain HepG2-HBV-Gluc

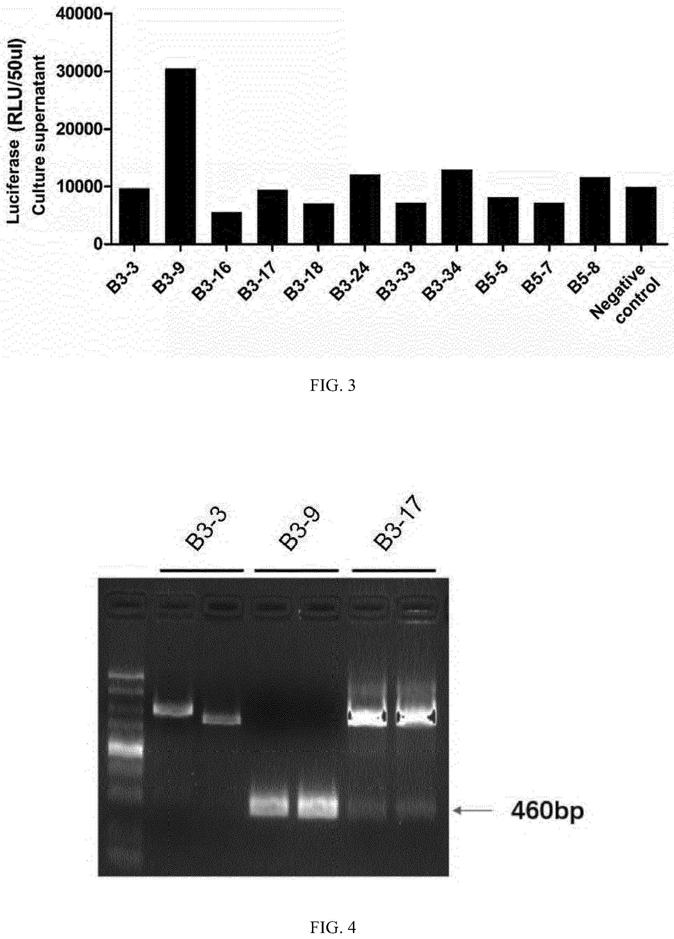

The expression level of Gaussia Luciferase varies due to the different insertion positions of gene fragments in different cell clones. In order to pick cell clones with high Gaussia Luciferase expression, it is necessary to test the expression of luciferase in the picked clones. Gaussia Luciferase Assay System kit (Promega, E2810) was used to detect Gaussia Luciferase. The supernatants from different cell clones were absorbed and mixed with the detection reagent in the same volume. After sufficient reaction, the fluorescence values were read out on a multi-well microplate luminescence detector (GLOMAX^{®}-96). The results were shown in FIG. 3. The horizontal axis represents different cell strains, and the vertical axis represents the fluorescence value.

As can be seen from FIG. 3, the monoclonal cell strain labeled as B3-9 produced the most fluorescent signals, indicating a greatest amount of cccDNA produced within the cell.

### 6. Induction and Identification of cccDNA Formation

The cell pellets of different cell clones were collected, and the total DNA in cells was extracted using a DNA extraction kit (Tiangen Biochemical Co., Ltd.) for PCR identification. The PCR system was shown in Table 3. The PCR procedure was shown in Table 4. The primer sequences in PrimerMix were as set forth in SEQ ID NO: 8 to SEQ ID NO: 9. The PCR identification results were shown in FIG. 4.

**Table 3**

| | |
|---|---|
| PrimeSTAR^{®} Max | 12.5 µL |
| Primer Mix | 1 µL |
| DNA | 1 µL |
| RNase free water | 8.5 µL |
| Total Volume | 25 µL |

**Table 4**

| | | | |
|---|---|---|---|
| Pre- denaturation | 94 °C | 5 min | |
| Denaturation | 94 °C | 30 s | 20 cycles |
| Annealing | 60 °C | 30 s | |
| Extension | 65 °C | 3 min 20 s | |
| Extension | 65 °C | 5 min | |
| | 4 °C | keep | |

As can be seen from FIG. 4, the monoclonal cell strain labeled as B3-9 produces cccDNA.

### 7. Test of Stability of Cell Lines with cccDNA Inhibitor CCC-0975

CCC-0975 is known to have an inhibitory effect on cccDNA, so this compound was used to test the effectiveness of stable cell lines to produce cccDNA. The specific procedure was as follows.

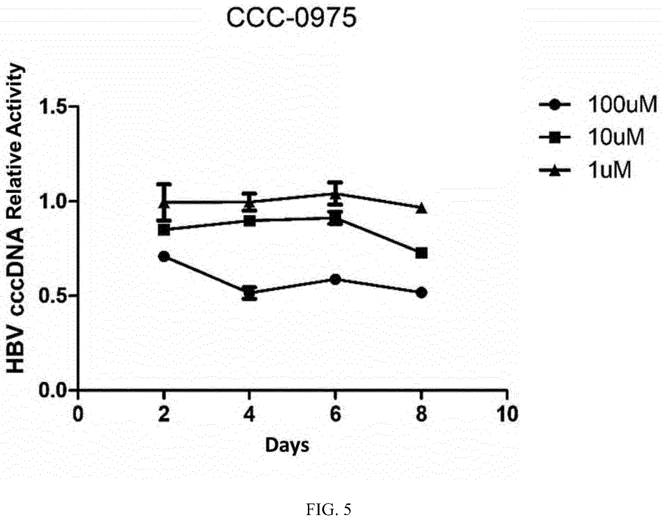

50,000 correctly screened cells (monoclonal cell strain labeled as B3-9) were grown in each well in a 96-well plate and cultured overnight in DMEM medium supplemented with 10% FBS. After 24 hours, the CCC-0975 compound at concentrations of 100 µM, 10 µM, and 1 µM was formulated in DMEM medium supplemented with 5% FBS. At 24 hr, 48 hr, 72 hr, and 96 hours after adding the compound, the culture medium supernatants were collected for measuring activity of the Gaussia Luciferase. The supernatants of different experimental groups were pipetted and mixed with the detection reagent in the same volume. After sufficient reaction, the fluorescence values were read out on a multi-well microplate luminescence detector (GLOMAX^{®}-96). The results are shown in FIG. 5. In FIG. 5, the horizontal axis represents different time points, and the vertical axis represents the ratio of the experimental group to the control group.

As can be seen from FIG. 5, CCC-0975 had an inhibitory effect on cccDNA, indicating that the cccDNA produced by the monoclonal cell strain labeled as B3-9 was active.

The technical features of the embodiments described above may be arbitrarily combined. For the sake of brevity of description, not all possible combinations of the technical features in the aforementioned embodiments are described. However, as long as there is no contradiction between the combinations of these technical features, all should be considered as the scope of this specification.

The above embodiments only represent several examples of the present disclosure, and the description thereof is more specific and detailed. but it should not be construed as restricting the scope of the present disclosure. It should be understood that, the applications of the present disclosure are not limited to the above-described examples, and those skilled in the art can make modifications and changes in accordance with the above description, all of which are within the protection scope of the present disclosure. Therefore, the protection scope of the present disclosure shall be defined by the appended claims.

## Claims

1. A nucleic acid for preparing HBV cccDNA comprising a first segment and a second segment linked to the first segment, wherein the first segment and the second segment are each transcribed independently; the first segment comprises a Core gene encoding a core protein, a Pol gene encoding an HBV polymerase and an X gene encoding an HBx; the second segment comprises a nucleic acid fragment corresponding to a pregenomic RNA and a nucleic acid fragment corresponding to a start codon; the nucleic acid fragment corresponding to the start codon is positioned at the 3' end of a transcription direction of the nucleic acid fragment corresponding to the pregenomic RNA; only one nucleic acid fragment corresponding to the start codon is present in the second segment; and the nucleic acid contains no expression frame of S gene encoding an HBs protein.

2. The nucleic acid according to claim 1, wherein the second segment further comprises a reporter gene, and the reporter gene, the nucleic acid fragment corresponding to the pregenomic RNA and the nucleic acid fragment corresponding to the start codon are sequentially linked in a transcription direction of the second segment.

3. The nucleic acid according to claim 2, wherein the reporter gene is at least one selected from the group consisting of a luciferase gene, a chloramphenicol acetyltransferase gene, a beta-galactosidase gene, a secretory human placental alkaline phosphatase gene, and a green fluorescent protein gene.

4. The nucleic acid according to any one of claims 1 to 3, wherein the Core gene has a nucleotide sequence as set forth in SEQ ID NO: 1.

5. The nucleic acid according to any one of claims 1 to 4, wherein the Pol gene has a nucleotide sequence as set forth in SEQ ID NO: 2.

6. The nucleic acid according to any one of claims 1 to 5, wherein the X gene has a nucleotide sequence as set forth in SEQ ID NO: 3.

7. The nucleic acid according to any one of claims 1 to 6, wherein the first segment further comprises an antibiotic resistance gene, and the antibiotic resistance gene is linked to the Core gene via a nucleic acid fragment encoding a 2A peptide.

8. The nucleic acid according to claim 7, wherein the antibiotic resistance gene has a nucleotide sequence as set forth in SEQ ID NO: 5.

9. The nucleic acid according to claim 1, wherein the first segment has a nucleotide sequence as set forth in SEQ ID NO: 6.

10. The nucleic acid according to claim 1, wherein the second segment has a nucleotide sequence as set forth in SEQ ID NO: 7.

11. A vector for preparing HBV cccDNA comprising the nucleic acid for preparing HBV cccDNA of any one of claims 1 to 10.

12. A host cell comprising the vector for preparing HBV cccDNA of claim 11.

13. A method for preparing HBV cccDNA comprising:
transfecting a host cell with a vector for preparing the HBV cccDNA of claim 11 and then culturing the host cell to construct a stable cell line for preparing HBV cccDNA.

14. The method according to claim 13, wherein the host cell is HepG2, Huh7, a cell line derived from HepG2 or a cell line derived from Huh7.

## Patentansprüche

1. Nukleinsäure zur Herstellung von HBV-cccDNA, umfassend ein erstes Segment und ein an das erste Segment gebundenes zweites Segment, wobei das erste Segment und das zweite Segment jeweils unabhängig voneinander transkribiert werden; wobei das erste Segment ein Core-Gen, das für ein Core-Protein codiert, ein Pol-Gen, das für eine HBV-Polymerase codiert, und ein X-Gen, das für ein HBx codiert, umfasst; wobei das zweite Segment ein Nukleinsäurefragment, das einer prägenomischen RNA entspricht, und ein Nukleinsäurefragment, das einem Startcodon entspricht, umfasst; wobei das dem Startcodon entsprechende Nukleinsäurefragment am 3'-Ende der Transkriptionsrichtung des der prägenomischen RNA entsprechenden Nukleinsäurefragments positioniert ist; wobei im zweiten Segment nur ein dem Startcodon entsprechendes Nukleinsäurefragment vorhanden ist; und wobei die Nukleinsäure keinen Expressionsrahmen des S-Gens enthält, das für ein HBs-Protein codiert.

2. Nukleinsäure nach Anspruch 1, wobei das zweite Segment ferner ein Reportergen umfasst, und das Reportergen, das der prägenomischen RNA entsprechende Nukleinsäurefragment und das dem Startcodon entsprechende Nukleinsäurefragment in Transkriptionsrichtung des zweiten Segments sequenziell miteinander verbunden sind.

3. Nukleinsäure nach Anspruch 2, wobei das Reportergen mindestens eines ist, ausgewählt aus der Gruppe bestehend aus einem Luciferase-Gen, einem Chloramphenicol-Acetyltransferase-Gen, einem Beta-Galactosidase-Gen, einem sekretorischen humanen plazentaren alkalischen Phosphatase-Gen und einem Gen für grün fluoreszierendes Protein.

4. Nukleinsäure nach einem der Ansprüche 1 bis 3, wobei das Core-Gen eine Nukleotidsequenz aufweist, wie in SEQ ID NO: 1 angegeben.

5. Nukleinsäure nach einem der Ansprüche 1 bis 4, wobei das Pol-Gen eine Nukleotidsequenz aufweist, wie in SEQ ID NO: 2 angegeben.

6. Nukleinsäure nach einem der Ansprüche 1 bis 5, wobei das X-Gen eine Nukleotidsequenz aufweist, wie in SEQ ID NO: 3 angegeben.

7. Nukleinsäure nach einem der Ansprüche 1 bis 6, wobei das erste Segment ferner ein Antibiotikaresistenzgen umfasst und das Antibiotikaresistenzgen über ein Nukleinsäurefragment, das ein 2A-Peptid codiert, mit dem Core-Gen verbunden ist.

8. Nukleinsäure nach Anspruch 7, wobei das Antibiotikaresistenzgen eine Nukleotidsequenz aufweist, wie in SEQ ID NO: 5 angegeben.

9. Nukleinsäure nach Anspruch 1, wobei das erste Segment eine Nukleotidsequenz aufweist, wie in SEQ ID NO: 6 angegeben.

10. Nukleinsäure nach Anspruch 1, wobei das zweite Segment eine Nukleotidsequenz aufweist, wie in SEQ ID NO: 7 angegeben.

11. Vektor zur Herstellung von HBV-cccDNA, umfassend die Nukleinsäure zur Herstellung von HBV-cccDNA nach einem der Ansprüche 1 bis 10.

12. Wirtszelle, die den Vektor zur Herstellung von HBV-cccDNA nach Anspruch 11 umfasst.

13. Verfahren zur Herstellung von HBV-cccDNA, umfassend:
Transfizieren einer Wirtszelle mit einem Vektor zur Herstellung der HBV-cccDNA nach Anspruch 11 und anschließendes Kultivieren der Wirtszelle, um eine stabile Zelllinie zur Herstellung von HBV-cccDNA zu konstruieren.

14. Verfahren nach Anspruch 13, wobei die Wirtszelle HepG2, Huh7, eine von HepG2 abgeleitete Zelllinie oder eine von Huh7 abgeleitete Zelllinie ist.

## Revendications

1. Acide nucléique pour préparer un ADNccc de VHB comprenant un premier segment et un second segment lié au premier segment, dans lequel le premier segment et le second segment sont chacun transcrits indépendamment ; le premier segment comprend un gène Core encodant une protéine noyau, un gène Pol encodant une polymérase de VHB et un gène X encodant une HBx ; le second segment comprend un fragment d'acide nucléique correspondant à un ARN prégénomique et un fragment d'acide nucléique correspondant à un codon de départ ; le fragment d'acide nucléique correspondant au codon de départ est positionné à l'extrémité terminale 3' d'une direction de transcription du fragment d'acide nucléique correspondant au ARN prégénomique ; seulement un fragment d'acide nucléique correspondant au codon de départ est présent dans le second segment ; et l'acide nucléique ne contient aucun cadre d'expression de gène S encodant une protéine HBs.

2. Acide nucléique selon la revendication 1, dans lequel le second segment comprend en outre un gène rapporteur, et le gène rapporteur, le fragment d'acide nucléique correspondant au ARN prégénomique, et le fragment d'acide nucléique correspondant au codon de départ sont séquentiellement liés dans une direction de transcription du second segment.

3. Acide nucléique selon la revendication 2, dans lequel le gène rapporteur est au moins un sélectionné parmi le groupe constitué de : un gène de luciférase, un gène de chloramphénicol acétyltransférase, un gène de bêta-galactosidase, un gène de phosphatase alcaline placentaire humaine sécrétoire, et un gène de protéine fluorescente verte.

4. Acide nucléique selon l'une quelconque des revendications 1 à 3, dans lequel le gène Core a une séquence nucléotidique telle que présentée dans SEQ ID n° : 1.

5. Acide nucléique selon l'une quelconque des revendications 1 à 4, dans lequel le gène Pol a une séquence nucléotidique telle que présentée dans SEQ ID n° : 2.

6. Acide nucléique selon l'une quelconque des revendications 1 à 5, dans lequel le gène X a une séquence nucléotidique telle que présentée dans SEQ ID n° : 3.

7. Acide nucléique selon l'une quelconque des revendications 1 à 6, dans lequel le premier segment comprend en outre un gène de résistance aux antibiotiques, et le gène de résistance aux antibiotiques est lié au gène Core par l'intermédiaire d'un fragment d'acide nucléique encodant un peptide 2A.

8. Acide nucléique selon la revendication 7, dans lequel le gène de résistance aux antibiotiques a une séquence nucléotidique telle que présentée dans SEQ ID n° : 5.

9. Acide nucléique selon la revendication 1, dans lequel le premier segment a une séquence nucléotidique telle que présentée dans SEQ ID n° : 6.

10. Acide nucléique selon la revendication 1, dans lequel le second segment a une séquence nucléotidique telle que présentée dans SEQ ID n° : 7.

11. Vecteur pour préparer un ADNccc de VHB comprenant l'acide nucléique pour préparer un ADNccc de VHB de l'une quelconque des revendications 1 à 10.

12. Cellule hôte, comprenant le vecteur pour préparer un ADNccc de VHB de la revendication 11.

13. Procédé pour préparer un ADNccc de VHB, comprenant :
la transfection d'une cellule hôte avec un vecteur pour préparer le ADNccc de VHB de la revendication 11 et puis la mise en culture de la cellule hôte pour construire une lignée cellulaire stable pour préparer un ADNccc de VHB.

14. Procédé selon la revendication 13, dans lequel la cellule hôte est HepG2, Huh7, une lignée cellulaire dérivée de HepG2, ou une lignée cellulaire dérivée de Huh7.
